# EUROPEAN PATENT APPLICATION

(11) **EP 4 529 889 A1**
(43) Date of publication of application: **02.04.2025**
(21) Application number: 24203138.3
(22) Date of filing: 27.09.2024
(51) Int. Cl.: A61F 2/36, A61F 2/30

(54) **RESURFACING HEAD FOR BALL AND SOCKET JOINT**

(30) Priority: 29.09.2023 US 202363541391 P
(71) Applicant: Howmedica Osteonics Corporation, Mahwah, New Jersey 07430 (US)
(72) Inventor: HANSON, Keenan Michael, New York, NY 10974 (US); MULLEN, Lewis, Mahwah, NJ 07430 (US); SCHMIDT, Walter, Rockaway, NJ 07866 (US); KREBS, Viktor Erik, Rocky River, OH 44116 (US); MONT, Michael, Owings Mill, MD 21117 (US)
(74) Representative: Keller Schneider Patentanwaltsgesellschaft mbH

(57) **Abstract**

A femoral head resurfacing implant (100) includes a head having a rim (130) separating an outer surface (120) of the head from an inner surface (122) of the head. The head has a shape such that a first plane (85) extends through three separate locations along a length of a rim of the head. The implant also includes a stem (125) extending from the inner surface of the head to a tip (127) that is interior to an opening (124) of the implant. Additionally, the stem is entirely on the first side of the first plane. The stem may be configured to be implanted within the natural bone of a patient at an angular offset designed to preserve the integrity of the preexisting bone structure.

## Description

### CROSS-REFERENCE TO RELATED APPLICATION

This application claims the benefit of U.S. Provisional Patent App. No. 63/541,391 filed September 29, 2023, the disclosure of which is hereby incorporated by reference herein in its entirety.

### BACKGROUND OF THE INVENTION

Ball and socket joints of a patient may degenerate due to a variety of etiologies, such as arthritis, or may fracture or deteriorate due to injury from a traumatic event. In such circumstances, an orthopedic procedure may be necessary to restore function and stability to the joint. Orthopedic procedures in a ball and socket joint of the human body typically involve replacement of at least some of the natural joint through the use of prosthetic devices to supplement or replace components of the joint.

Orthopedic procedures in ball-and-socket joints may be broadly divided between total joint replacement and resurfacing procedures. While resurfacing procedures provide many benefits relative to total joint replacement, such as preservation of more original bone, a higher probability of natural load transfer, reduced stress shielding and associated complications through such improved natural load transfer, and reduced risk of dislocation, such procedures may not be a viable option in many instances. Difficulties include, for example, achieving sufficient anchorage of a resurfacing implant on the ball of the ball and socket j oint, and further, providing an implant that, when implanted, can be expected to last for a long duration under expected wear without the need for a revision surgery.

Thus, there is a need for improvements in the implants and associated methods that restore function in ball-and-socket joints within the body.

### BRIEF SUMMARY OF THE INVENTION

In one aspect, the present disclosure relates to a resurfacing implant for a ball-shaped bone in a ball and socket j oint. In one embodiment, an implant includes a femoral head component with a convex outer surface sized and configured to be received within an acetabulum of a patient. The femoral head component may further include a concave inner surface sized and configured to be received on a prepared femoral head of the patient. The implant may include a femoral head stem for anchorage of the implant to the prepared head of the femur. The femoral head stem may be positioned on the concave inner surface and have a length that is sized and angled such that the stem does not extend into a tensile region of the neck and head of the patient's femur. The femoral prothesis may further be positioned and have a length that is sized and angled such that the implant does not extend into an intersection of the tensile region and a portion of cortical bone. The implant may be implanted onto the femoral head at an angular offset from the axis of the femoral neck of the patient.

In a first embodiment of the implant, the implant includes a femoral head resurfacing prothesis with a rim separating an outer surface of the head from an inner surface of the head. The rim may be shaped such that a first plane may pass through three separate locations along a length of the rim such that an entirety of the head is on a first side of the first plane. The implant also includes a stem extending from the inner surface of the head that may be configured to be received within an opening of a femoral head of a femur, the stem being entirely on the first side of the first plane. Variations of these embodiments of the implant may include an inner surface configured to contain a spherical interface region, a cylindrical region, and an elevated, circular ridge surrounding the stem of the implant. Additional variations of the implant may include a stem oriented at an angle relative to a first axis, the first axis passing through a pole of the head and a center of an opening defined by the rim. The base of the stem of the implant may be offset from the first axis. Further, the stem may be oriented at an angle within a range from 15 to 45 degrees relative to the first axis, or at an angle within a range from 25 to 35 degrees relative to the first axis. In certain embodiments, a length of the stem is less than or equal to 60% of the outer diameter of the implant.

In a second embodiment of the implant, the implant includes a femoral head resurfacing prosthesis with a head having a rim separating an outer surface of the head from an inner surface of the head, a stem extending from the inner surface toward an opening of the head defined by the rim, in which a central axis of the head passes through a center of the opening and a pole of the head. The stem has an elongate dimension oriented at an angle in a range from 5 degrees to 20 degrees relative to the central axis of the head. Further, the elongate dimension of the stem is less than 65% of an outer diameter of the head. In some variations, the implant may include a head with a base component and an articulating surface component attachable to the base component, with the inner surface on the base component and the outer surface on the articulating surface component.

Further variations may include a modular system including a head with an inner surface with an elevated, circular ridge surrounding the stem. The stem may have elongated dimensions and the stem may be oriented at an angle in a range from 10 degrees to 15 degrees relative to the central axis of the head. The elongate dimension of the stem may further fall within a range from 50% to 60% of the outer diameter of the head.

In another embodiment, the present disclosure may include a modular kit with a femoral head resurfacing implant, as described supra, further including a second articulating surface component different from the first articulating surface component.

In another aspect, the present disclosure relates to a method of implanting a resurfacing implant in a hip joint of a patient. In a first embodiment, the method may include one or more of the following steps: resecting a first portion of a head of the femur such that a second portion of the head remains; retrieving a femoral head implant, the femoral head implant including a head and a stem, the head including a convex outer surface and a concave inner surface and the stem extending from the concave inner surface to a free end; advancing the femoral head implant into the second portion of the head of the femur; and securing the femoral head resurfacing implant onto the second portion of the head such that when the concave inner surface contacts the second portion of the head, the stem lies outside of a tensile stress region of the femur. The femoral head resurfacing implant may be fixed to the femoral head such that a central axis of the femoral head resurfacing implant is positioned at an angle relative a central longitudinal axis of a femoral neck of the femur, the central axis passing through a center of an opening of the head and a pole of the head. In a variation of the method of this embodiment, the step of securing the femoral head resurfacing implant onto the second portion may include positioning the head such that when the concave inner surface contacts the second portion of the head, the implant lies outside of the intersection of the tensile region and the portion of cortical bone of the femur.

Further in variations of the method of the first embodiment, the femoral head may be secured to the second portion of the head, with the stem is oriented at an angle ranging from 15 degrees to 45 degrees relative to an axis of a femoral neck of the femur, or oriented at an angle ranging from 25 degrees to 35 degrees relative to an axis of a femoral neck of the femur. Further, the femoral head may be secured to the second portion of the head. The base of the stem may abut the concave inner surface of the head with an offset from a central axis of the head passing through a center of an opening of the head and a pole of the head.

In a second embodiment, a method of implanting a modular femoral head resurfacing system in a patient within the present disclosure may include: resecting a first portion of a head of the femur within the patient such that a second portion of the head remains; retrieving a resurfacing system including a base component and an articulating surface component; disposing the base component onto the second portion of the head such that a stem of the base component is oriented at an angle relative to a central axis of the femoral neck; securing the base component to the femur; and attaching the articulating surface component onto the base component. Further, the stem may be prevented from extending into the tensile region of the femur during implantation and fixation. The femoral head implant may further be prevented from extending into the intersection of the tensile region and the portion of cortical bone. Additionally, the femoral head may be secured to the second portion of the head, with the stem oriented at an angle ranging from 15 degrees to 45 degrees relative to an axis of a femoral neck of the femur, or oriented at an angle ranging from 25 degrees to 35 degrees relative to an axis of a femoral neck of the femur.

### BRIEF DESCRIPTION OF THE DRAWINGS

The present disclosure will become more fully understood from the detailed description and accompanying drawings, wherein:
FIG. 1A is a perspective view of the femoral head prosthesis according to an embodiment of the present disclosure;
FIG. 1B is a top-down view of the femoral head prosthesis of FIG. 1A;
FIG. 2 is a cross-sectional view of the femoral head prosthesis of FIG. 1 taken along a midline thereof;
FIG. 3 is a cross-sectional view of a modular implant system according to an embodiment of the present disclosure, taken along a midline thereof;
FIG. 4 is a side view of a femur according to a step of a method according to one embodiment of the present disclosure;
FIG. 5 is a side view of the femur of FIG. 4 according to another step in the method; and
FIG. 6 is a side view of the femur of FIG. 4 according to yet another step in the method.

### DETAILED DESCRIPTION

As used herein in reference to an implant, e.g., femoral head, the term "superior" refers to a portion of the implant nearer a patient's head while the term "inferior" refers to a portion of the implant nearer the patient's feet when the implant is implanted in an intended position and orientation in the body of the patient. As with the terms "superior" and "inferior," the term "anterior" refers to a portion of the implant nearer the front of the patient, the term "posterior" refers to a portion of the implant nearer the rear of the patient, the term "medial" refers to a portion of the implant nearer the mid-line of the patient, and the term "lateral" refers to a portion of the implant farther away from the mid-line of the patient. Additionally, the term "leading" refers to a portion of the implant that is inserted into the patient ahead of the remainder of the implant while conversely, the term "trailing" refers to a portion of the implant closest to an inserter instrument and is the last part of the implant inserted into the patient. Additionally, as used herein, the terms "about," "approximately," "generally," and "substantially" are intended to mean that slight deviations from absolute are included within the scope of the term so modified.

In one aspect, the present disclosure relates to a resurfacing implant for a ball-and-socket j oint in the human body. While specific embodiments of the present disclosure are described with respect to a proximal femur at a hip joint, it should be appreciated that the principles of the present disclosure are also contemplated for use in the shoulder.

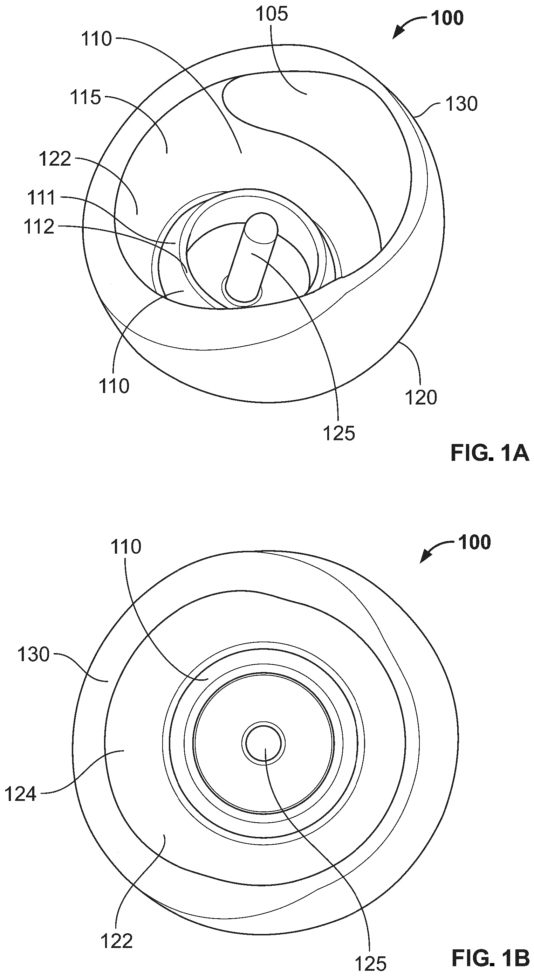

One embodiment of a femoral head resurfacing prosthesis is illustrated in FIGs. 1A-2 and is referred to as a prosthesis 100.

The prosthesis 100 may be made of various materials. Specifically, non-exhaustive examples of metallic materials that may be used are titanium and titanium alloys, stainless steel, cobalt chrome and special high-strength alloys. In alternative embodiments, the prosthesis may be comprised of other materials, such as ceramics and plastics. Non-exhaustive examples of plastics that may be used include ultra-high molecular weight polyethylene (UHMWPE), polytetrafluoroethylene (PTFE), polyetheretherketone (PEEK), highly cross-linked polyethylene, and composite such as a PEEK titanium composite. Non-exhaustive examples of ceramics that may be used include alumina, zirconia and zirconia toughened alumina (ZTA).

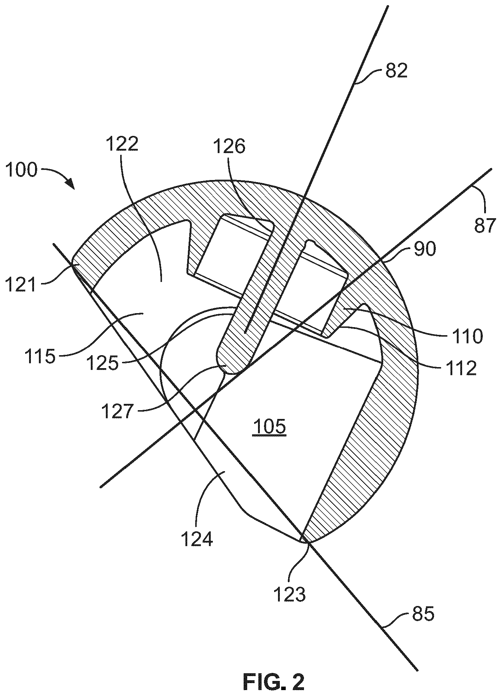

The prosthesis 100 includes a convex outer surface 120 sized and configured to be received within an acetabulum of a patient. In some variations, the convex outer surface 120 may be spherical in shape to mimic the inner surface of the acetabulum of a patient. Opposite the convex outer surface, the prosthesis 100 includes a concave inner surface 122 sized and configured to be received on a resected or otherwise prepared femoral head of the patient. As shown in FIGs. 1 and 2, the concave inner surface includes two surface regions: a cylindrical interface region 105 and a spherical interface region 115. The varied contours of the interface regions of the inner surface 122 of the prosthesis 100 correspond to a femoral head that is prepared to receive the prosthesis while also allowing for variations so that the prosthesis may be engaged at various angular offsets relative to the femur. Specifically, the prothesis 100 can be implanted onto the patient's natural bone at an angular offset from the axis of the femoral neck 12 of the patient. The outer surface 120 and the inner surface 122 are separated by a rim 130 around an opening 124 of the prothesis 100. The rim 130 may vary in width along the perimeter, as shown in FIG. 1B. A variance of the rim width may be a function of the type of surface region the rim 130 abuts at a given location on the rim when the prosthesis is implanted on a femoral bone.

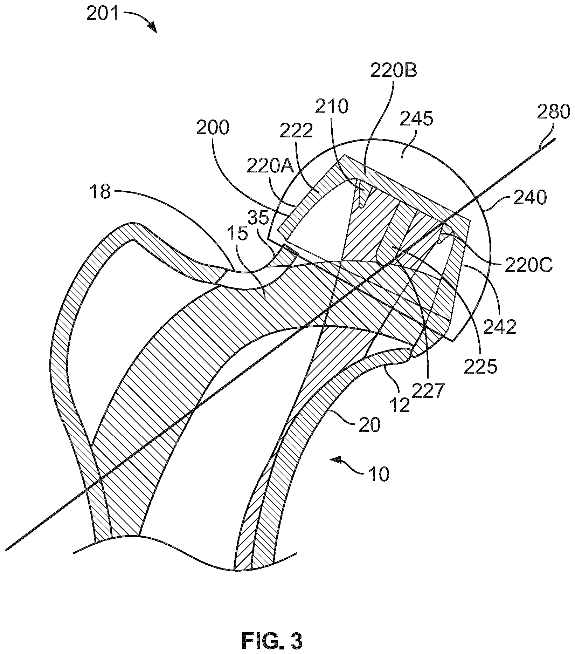

The prosthesis 100 also includes a femoral head stem 125 that extends from a base 126 at the inner surface 122 to a tip 127, as shown in FIGs. 1A-B and 2. The inclusion of the femoral head stem may aid in the anchorage of the prosthesis 100 to a prepared head of the femur bone. A length dimension of the head stem 125 may be in a range from about 40% to about 65% of an outer diameter of the prosthesis 100. In the depicted embodiment, the stem is shown as having a length approximately 40% that of the diameter, though such dimension is not limiting. In other examples, the length of the head stem may be a value less than 40% of the outer diameter of the prosthesis. In the contemplated embodiments, a guiding principle for the stem length is that it will remain outside of or at least largely outside of an intersection of the tensile region 15 and compressive region 20 in the proximal femur. Further, in the contemplated embodiments, the prosthesis, including the stem, is configured for implantation onto the proximal femur such that it does not disturb and is outside of the intersection 18 of the tensile region 15 and the portion of cortical bone in the proximal femur, as shown in FIG 3. Further, the preparation of the femur to receive the prothesis does not interfere with the bone and tissue in the region of the intersection. In this way, embodiments with a universal prosthesis design may have a stem sized to avoid a location of intersecting forces that would be expected in a variety of patient femurs. In other embodiments, the prosthesis may be patient specific and designed with a view to tensile region and compressive force region in the femur of the patient receiving the prosthesis. Additionally, the length of the head stem 125 may be such that the tip 127 is within an internal cavity of the prosthesis 127, between the inner surface 122 and a rim plane 85. The rim plane 85 generally corresponds to the rim 130 of the prosthesis, and for prosthesis 100, passes through a rim edge at a medial side 123 of the rim and a rim edge at a lateral side 121 of the rim, again shown in FIG 2. Further, a location of a base 126 of the head stem and a length of the head stem 125 may be such that the tip 127 is located proximate a central axis 87 of the prosthesis defined through a center of an opening 124 of the prosthesis and a pole 90 of the prosthesis, also approximately perpendicular to the rim plane 85, as shown in FIG. 2. As will be described in greater detail below, such stem dimensions minimize an extent to which the stem is disposed within regions of the bone expected to be in tension when the prosthesis is implanted.

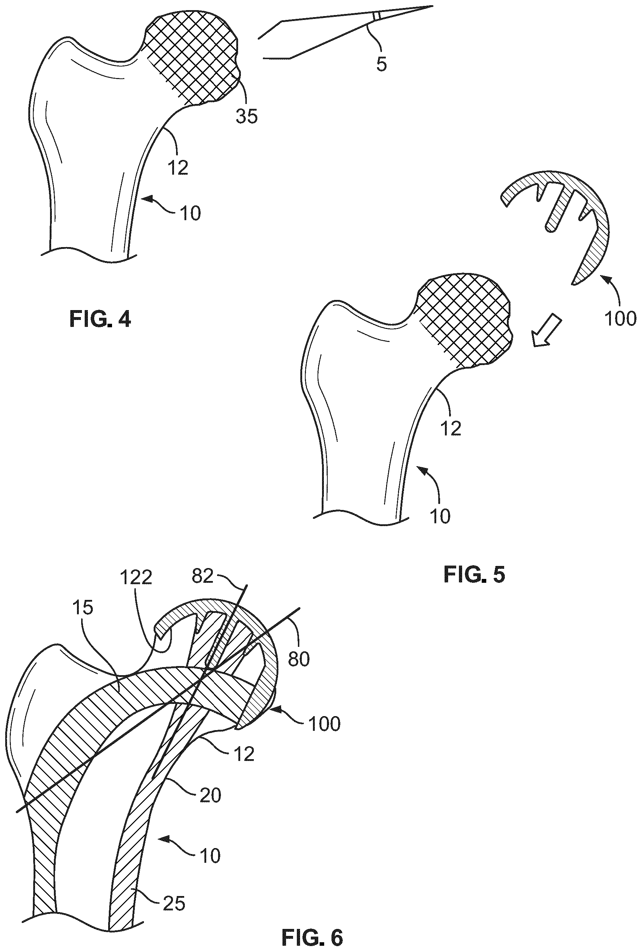

As shown in FIG. 2, an elongate dimension of the head stem 125 is at an angle relative to the central axis 87 of the prosthesis. In the depicted embodiment, this angle is approximately 15 degrees, though other angles are contemplated. Further, such angle may ultimately be measured based on a position of the prosthesis when implanted on a femoral head, as shown in FIG. 6 and discussed in greater detail below. It should be appreciated that in some examples, a central longitudinal axis of the head stem 125 of the prosthesis 100 may be at an approximately 25-30 degree angle relative to a central axis 80 of a native femoral neck 12 when implanted in a patient, as shown in FIG. 6. However, such angulation is merely illustrative and the angle of the head stem may be varied. In particular, such angle may be any specific value in a range from approximately 15 degrees to approximately 45 degrees. For instance, the angle between the central longitudinal axis of the head stem and the neck axis 80 when the prosthesis is implanted in a femur may be 15, 20, 25, 30, 35, 40 or 45 degrees. A location of the base 126 relative to the pole 90, i.e., an offset, may also be varied to suit a shape and characteristics of the femur receiving the prosthesis. It should be appreciated that determinations of one or more of a base location of the head stem relative to the pole of the prosthesis, the angle of the stem from the base, and the length of the stem may take into account the compression and tension regions in the native femoral neck 12 and head. Specifically, the natural femur of a patient includes sub-surface regions crucial for load transfer through the bone. These regions within the natural femur include the tensile region 15 and the compressive region 20, and the portion of cortical bone as shown in FIG. 6. The prothesis is configured to engage the natural femur in a manner that minimizes passage of the stem into the tensile region of the natural bone. The prothesis may further be configured to engage the natural femur in a manner that minimizes passage of the implant into the intersection 18 between the tensile region and the portion of cortical bone of the natural bone.

The prothesis 100 also includes an inner ring protrusion 110 extending from the inner surface 122 and spaced apart from and surrounding the femoral stem component 125, as shown in FIG. 1. In some examples such as prosthesis 100, the base 126 of the stem is at a center of the inner ring protrusion 110, as best shown in FIG. 1B. The inner ring protrusion 110 may enhance anchorage of the prosthesis 100 to bone and is optional such that some variations of the prosthesis may simply have a stem on the inner surface without the ring protrusion. The inner ring protrusion 110 may include a tapered profile such that an edge 112 is narrower than a base of the ring protrusion. Side surfaces of the ring protrusion may be flat or may have a patterned contour. For instance, an outer side surface 111 of the inner ring 110 may be frustoconical as shown in FIG. 1, spherical or cylindrical. The inner ring protrusion 110 may, in some examples, aid in implantation of the prosthesis 100 into the prepared head of the natural femur such that it is embedded into the bone in a circumference surrounding the stem component 125. Examples of such manner of implantation may include, but are not limited to, cement fixation, press-fit implantation, taper fit implantation, screw fixation, or other means.

Further, in variations of the prosthesis, the cylindrical interface region may be a greater or lesser proportion of the inner surface than in the prosthesis 100. In still further variations, the inner surface may be entirely defined by a cylindrical interface region 105 or entirely defined by a spherical interface region 115. The varied contours of the interface regions of the inner surface 122 of the prosthesis 100 may be shaped to mimic the native shape of the natural femoral head or the modified shape of the femoral head based on an expected resection of the femoral head surface, and as such, a determination of a surface contour of the prosthesis may be a function of the size and shape of the proximal femur, the physical condition of the proximal femur, and/or the angular offset sought for the positioning of the prosthesis, among other similar considerations.

The prosthesis 100 may be varied in many ways. For example, in variations of embodiments where the prosthesis includes a head stem, the head stem may have an elongate and/or cross-sectional shape different from head stem 125 and may include a series of teeth, other anchorage promoting surfaces or other features that ease the process of securement of the prosthesis to bone. In some embodiments, a fixation structure other than a head stem may be included on the prosthesis. Other fixation structures may include, for example, pins, screws, plates, and intramedullary nails or rods within the implant disclosed herein for the purpose of fixation to the patient's natural bone. Further embodiments may include a head stem shaped for fusing with the natural bone of the patient. Non-exhaustive examples of fixation structures include a head stem with interruptions extending into the stem to promote bone ingrowth. Further examples include stems with rough and uneven surface for promotion of bone ingrowth. Other examples may include a stem with a threaded outer surface in the configuration of a corkscrew for fixation into the natural bone. The body of the stem may taper into a tip that is narrower than the portion of the stem extending from the femoral head. In still further examples, the stem may be thicker or thinner relative to the head diameter than a thickness of the stem relative to the head diameter of prosthesis 100. In some of the contemplated embodiments, the prosthesis is monolithic such that the stem and the head are formed integrally. In other embodiments, the stem may be formed separate from the head and may be fixedly secured to the head. Such fixation may be through welding, for example.

In another embodiment of the present disclosure, a prosthesis in the form of a base component 200 may be part of a modular implant system 201 as shown in FIG. 3. System 201 may be used for head resurfacing in a ball and socket joint. For system 201, reference numerals in the 200-series of reference numerals refer to like elements in the 100-series of reference numerals, unless otherwise indicated. As shown in FIG. 3, modular implant system 201 includes a base component 200 and an articulating surface component 240 adapted to be received on the base component 200. Articulating surface component 240 is shown in phantom in FIG. 3 so that the base component 200 is visible together with the articulating surface component.

Turning initially to base component 200, base component includes a body with an outer side and a bone-facing inner side 222 opposite the outer side. The outer side includes multiple outer surfaces 220A, 220B, 220C, each being generally planar and at angles relative to each other as shown in FIG. 3. In variations, an exact contour and length of each of the outer surfaces may vary to the extent that such variations may still accommodate the receipt of a prosthetic femoral head similar to articulating surface component 240. The inner side 222 may include various combinations of bone interface surfaces, such as those described for prosthesis 100. For example, part of the inner side may be cylindrical and part may be spherical. In the depicted embodiment, a polar region of the inner side from which the stem 225 extends is largely flat. Such shape may be advantageous in that less bone needs to be resected to accommodate the implant with such a surface when compared to an inner surface curved in multiple directions. In further embodiments, the polar region may be conical or spherical. Further, the design of the polar region and indeed other regions of the prosthesis may follow the natural form of the femoral head. The details of the type of surface included in the design may be a function of the specific surgery to be performed with consideration given to the extent of bone resurfacing to be performed.

As with prosthesis 100, base component 200 may include a stem 225 extending from a base at the inner surface to a tip 227 and an inner ring 210 surrounding the stem component 225 such that the stem extends from a center of the inner ring. As with head stem 125 in prosthesis 100, the stem 225 is angulated and offset in a manner that improves anchorage and longevity of the implant. In FIG. 3, base component 200 is shown as implanted on a resected femur 10, and as shown, a length and position of the stem 225 within the neck 12 of the femur is such that the stem is largely or entirely within a compressive region 20 of the bone, i.e., a region that experiences compressive forces during expected post-surgical wear. The inner ring 210 and the stem 225 may be varied in each of the ways described for such features in prosthesis 100 and may be configured to serve the same functions as described with respect to prosthesis 100. For example, a central longitudinal axis of the stem 225 may be at an approximately 25-30 degree angle relative to the neck axis 280 when implanted on the neck 12. However, such angulation is merely illustrative and the angle of the head stem may be varied. In particular, such angle may be any specific value in a range from approximately 15 degrees to approximately 45 degrees. For instance, the angle between the central longitudinal axis of the stem 225 when the prosthesis is implanted in a femur may be 15, 20, 25, 30, 35, 40 or 45 degrees relative to axis 280. Further, a length dimension of the stem 225 may be in a range from about 40% to about 65% of an outer diameter of the prosthesis 201. In other examples, the length of the stem may be a value less than 40% of the outer diameter of the prosthesis. Additionally, the variations of the prosthesis described for prosthesis 100 may also apply in the same manner to prosthesis 201 of system 200.

As to the prosthetic femoral head, referred to as the articulating surface component 240, such component 240 includes an outer side with a convex outer surface 245 and an inner side with inner surfaces 242. The convex outer surface 245 is configured to articulate within a cavity of an acetabulum of the patient and may be spherical in shape to complement the surface within the cavity. And, inner surfaces 242 complement outer surfaces 220A, 220B, 220C of base component 200 so that component 240 may be attached to base component 200. A press-fit or a press-fit taper assembly provides a means for attachment of articulating surface component 240 to base component 200 and is particularly well suited for assembly of modular configurations of those components. It should be appreciated, however, that attachment between an articulating surface component and a base component may be through threading, welding, snap locking, compression molding, or injection molding.

The head component 240 may be available in multiple offsets and sizes to mimic the natural shape and configuration of the patient's bone structure or to otherwise accommodate a desired leg length and/or offset of the reconstructed hip joint, or other joint as applicable. While system 201 depicts a single articulating surface component 240, it should be appreciated that kits are contemplated by the present disclosure, and in some embodiments, a kit may include a plurality of articulating surface components adapted to be received on a single base component. Among the plurality of articulating surface components, each one may have a different angulation relative to an attachment surface on the base component to produce a unique angulation, superior/inferior position on the femur, and/or rotational position on the femur and relative to the neck axis 280.

The head component 240 may be made of various materials. Specifically, non-exhaustive examples of metallic materials that may be used include titanium and titanium alloys, stainless steel, cobalt chrome and special high-strength alloys. In alternative embodiments, the prosthesis may be comprised of other materials, such as ceramics and plastics. Non-exhaustive examples of plastics that may be used are ultra-high molecular weight polyethylene (UHMWPE), polytetrafluoroethylene (PTFE), polyetheretherketone (PEEK), and highly cross-linked polyethylene, and other polymeric materials within the PAEK family. Non-exhaustive examples of ceramics that may be used include alumina, zirconia and zirconia toughened alumina (ZTA).

As described above, the various embodiments of the resurfacing prosthesis of the present disclosure may be configured to engage the patient's natural bone at various offsets and angles. Such versatility is advantageous in many ways. The natural femur of a patient includes sub-surface regions crucial for load transfer through the bone. These regions within the natural femur include the tensile region 15 and the compressive region 20. The angular offset facilitates implantation of the prosthesis 100 or system 201 onto the prepared femoral head such that an angle between the stem of the prosthesis and the neck axis facilitates load transfer through the bone as would occur naturally. In the depicted embodiments, and as shown in FIGS. 3 and 6 and described in greater detail elsewhere in the present disclosure, the head stem 125, 225 is received in a femoral head so that it is entirely within a compressive region 20 of the bone and does not enter a tensile region 15 or enter the intersection 18 of the tensile region and the portion of cortical bone. In this manner, disruption of the tensile region 15 and the intersection 18 between the tensile region and the portion of cortical bone is prevented. The emulation of a natural loading pattern in the femur is advantageous in that it may decrease stress induced bone remodeling and bone resorption. Further, decreased resorption may decrease the rate of periprosthetic fracture.

Moreover, the prostheses described herein are designed to be implanted within a patient's natural bone with only minimal bone resection and in this manner is advantageous in that a significant amount of the patient's existing bone is preserved. The preservation of key regions of the natural bone of the patient increases the available surgical options if future revision is necessary. This is because additional bone stock is maintained relative to procedures that involve total proximal femur replacement. Further, the bone stock preserved with the use of the contemplated prostheses may result in a stiffness and modulus in the femur that closely matches the native femur, improving patient feel.

In another aspect, the present disclosure relates to a kit with implants and/or implant components for use in surgery. In one embodiment, a kit includes two or more head resurfacing prostheses. In some examples of this embodiment, the two or more prostheses include prostheses that are different from each other. In some examples, the kit includes at least one single component prosthesis such as the prosthesis 100 and at least one two-component prosthesis 200. In some embodiments, a kit includes at least one base component of a two-component prosthesis and a plurality of articulating surface components. In further embodiments, a kit may include any combination of prostheses as contemplated by the present disclosure. In any one of the above embodiments, a kit may also include instrumentation 5 used for bone preparation as is shown in FIG. 4 and/or instrumentation for the implantation of the prosthesis. In some examples of the above embodiments, a kit may be accompanied by an instruction manual on how to perform one or more of the methods of using the contents of the kit.

The implants and components contemplated by the present disclosure may be manufactured by various methods such as additive manufacturing or various forms of conventional manufacturing. Examples of additive manufacturing techniques that may be utilized include Fused Deposition Modelling ("FDM"), Shape Deposition Manufacturing ("SDM"), Selective Laser Power Processing ("SLPP"), Direct Metal Laser Sintering ("DMLS"), Selective Laser Sintering ("SLS'), Selective Laser Melting ("SLM"), Selecting Heating Sintering ("SHS"), Electron Beam Melting ("EBM"), material jetting, binder jetting, or the like. Additional details of exemplary additive manufacturing methods are described in U.S. Pat. Nos. 7,537,664, 8,590,157, 8,728,387, 9.180,010 and 9,456,901, the disclosures of which are hereby incorporated by reference herein in their entireties. As to conventional manufacture, methods of manufacture may include injection molding, forging or investment casting, rough machining, and may be complemented by polishing and coating processes. Designs for the implants and components may be developed through the use of various modeling and simulation programs, such as, but not limited to, Computer Aided Design Software ("CAD") used to create vector-based graphics.

In another aspect, the present disclosure relates to a method of performing a bone resurfacing and restoration procedure at a hip joint.

In one embodiment shown in FIGs. 4-6, the method involves restoring a hip joint and in particular a femoral head of the hip joint using a resurfacing implant. In this method, the prothesis may be implanted into a patient's natural hip joint with minimal disturbance of the natural bone. A plan for the design of a particular shape, size and orientation of the prosthesis and stem may be prepared prior to surgery through evaluation of patient images such as x-ray images or CT-scans that indicate a condition of the bone to be operated upon. The patient images may be uploaded onto software to aid in such design process. The design of the stem angle and its position when implanted in the femoral head may be guided by the characteristics of the natural bone, including locations of compressive region, tensile region, and the portion of cortical bone in the neck 12 of the bone, as are shown, for example, in FIGS. 3 and 6. Upon finalization of a design for the prosthesis, the prosthesis may be manufactured. In some examples, the prosthesis may be manufactured according to the methods described elsewhere in the present disclosure.

While some preparation of the patient's natural femoral head is required, the implantation of the prosthesis 100, a resurfacing implant, does not require removal of the entirety of the natural femoral head of the patient or reaming of the proximal intramedullary canal of the neck 12 of the femur of the patient. To prepare a resected surface 35 of the femoral head to receive the prosthesis, the femoral head of the patient is initially resected via a robotic instrument, a manual technique, or a combination of both, as shown in FIG. 4. Examples of robotically controlled resection instruments used to resect the femoral head may include those described in U.S. Pat. App. Pub. No. 2020/0261232, assigned to Howmedica Osteonics Corp, the disclosure of which is hereby incorporated by reference herein in its entirety. Additional bone may then be removed to create a hole (not shown) through the head of the femur 10 for receipt of the stem 125 of the prosthesis. The prothesis 100 may then by implanted by placement of the prosthesis 100 onto the resected surface 35 so that inner surface 122 abuts the resected surface and the stem component 125 is received into the prepared hole, as shown in FIGs. 5 and 6. Fixation may be achieved in several ways including press fit, taper fit, screw fixation, or other means. Based on the shape of the prosthesis used, the stem may be inserted in a manner that causes minimal disruption to the tensile region 15 of the femur when the prosthesis 100 is implanted onto the femoral head, as shown in FIG. 6. Further, as shown in FIG. 3, the implant may be inserted in a manner that causes minimal disruption to the intersection of the tensile region and the portion of cortical bone of the femur when the prosthesis 100 is implanted onto the femoral head.

In another embodiment, the method of restoring the hip joint involves preparation of a femoral head and implantation of the prosthesis system 201 onto the femoral head. This method may be performed based on the same steps as described for the method using prosthesis 100, with additional steps involving the selection and attachment of the articulating surface component 240 onto the base component 200. The system may include head components 240 of various sizes and offsets. Trials of each head component 240 may be used to determine the most compatible size and offset for the patient. In one example, an initial head component is selected and secured to the base component 200 in a non-permanent manner. The size, fit, and offset may then be evaluated. If the selected the head component does not suit the planned revision of the hip j oint, the head component is removed and anew size is selected. The process may be repeated until an optimal head component 240 is identified.

Like the implantation of the prothesis 100, the system 201 may be implanted into a patient's natural hip joint with minimal impact to the natural bone. The femoral head or the patient's natural femur may initially be surfaced via a robotic, manual, or combination technique. Then, the base component 200 may be fixed to the prepared bone. The angle of the stem 225 of the base component 200 for implantation may be established to avoid interference with the tensile region 15 and/or with the intersection 18 between the tensile region and the portion of cortical bone in the neck 12 of the femur. Fixation of base component 200 into the bone may be achieved in several ways including, for example, press fit, taper fit, screw fixation, or other means. Subsequently, the selected femoral head component 240 may be applied to the base component 200 to complete the assembly of the prosthesis system 201.

The method of performing a bone resurfacing and restoration procedure may be varied in many ways. For example, design of the prosthesis may simply involve selection of an off the shelf size suitable for the patient at issue. In such cases, the prosthesis may be available in two or more sizes. Similarly, such universal prosthesis designs may include predetermined prosthesis shapes based on hip offset, anteversion and other geometric characteristics that may guide prosthesis selection. In such examples, the method of implantation following the selection may proceed as described for any one of the other described method embodiments. In still further examples, a method may begin with a prosthesis already available for use such that the design and manufacturing steps are not a part of the method of use. In other examples, the method may begin at the manufacturing step.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A femoral head resurfacing implant comprising:
a head having a rim separating an outer surface of the head from an inner surface of the head, wherein a first plane extends through three separate locations along a length of the rim; and
a stem extending from the inner surface of the head and being configured to be received within an opening of a femoral head of a femur, the stem being entirely on the first side of the first plane.

2. The femoral head resurfacing implant of claim 1, wherein an entirety of the head is on a first side of the first plane.

3. The femoral head resurfacing implant of any one of claims 1-2, wherein the inner surface is configured to contain a spherical interface region and a cylindrical region.

4. The femoral head resurfacing implant of any one of claims 1-3, wherein the inner surface is configured to contain an elevated, circular ridge surrounding the stem of the implant.

5. The femoral head resurfacing implant of any one of claims claim 1-4, wherein the stem is oriented at an angle relative to a first axis, the first axis passing through a pole of the head and a center of an opening defined by the rim.

6. The femoral head resurfacing implant of claim 5, wherein a base of the stem is offset from the first axis.

7. The femoral head resurfacing implant of claim 5, wherein the stem is oriented at an angle ranging from 15 to 45 degrees relative to the first axis.

8. The femoral head resurfacing implant of claim 5, wherein the stem is oriented at an angle ranging from 25 to 35 degrees relative to the first axis.

9. The femoral head resurfacing implant of claim 5, wherein the stem has an elongate dimension oriented at an angle in a range from 5 degrees relative to the first axis of the head.

10. The femoral head resurfacing implant of any one of claims 1-9, wherein the length of the stem is not greater than 60% of the outer diameter of the implant.

11. The femoral head resurfacing implant of any one of claims 1-10, wherein the head further comprises a base component and an articulating surface component attachable to the base component.

12. The femoral head resurfacing implant of any one of claim 11, wherein the inner surface is located on the base component.

13. The femoral head resurfacing implant of any one of claims 11-12, wherein the outer surface is located on the articulating surface component.

14. The femoral head resurfacing implant of any one of claims 1-13, further comprising an inner ring protrusion extending from an inner surface of the head, the inner ring protrusion being spaced apart from and surrounding the stem.
